(19) Europäisches Patentamt
European Patent Office
Office européen des brevets

(11) **EP 3 749 178 B1**

(12) **EUROPEAN PATENT SPECIFICATION**

(45) Date of publication and mention
of the grant of the patent:
**02.02.2022   Bulletin 2022/05**

(21) Application number: **19713187.3**

(22) Date of filing: **04.02.2019**

(51) International Patent Classification (IPC):
*A61B 5/00* (2006.01)        *A61B 5/08* (2006.01)

(52) Cooperative Patent Classification (CPC):
**A61B 5/08; A61B 5/0075; A61B 5/4875;**
A61B 2562/046

(86) International application number:
**PCT/IB2019/050868**

(87) International publication number:
**WO 2019/150333 (08.08.2019 Gazette 2019/32)**

(54) **A METHOD OF MEASURING PLEURAL EFFUSION**

VERFAHREN ZUR MESSUNG EINES PLEURAERGUSSES

PROCÉDÉ DE MESURE D'ÉPANCHEMENT PLEURAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **05.02.2018   PL 42451418**

(43) Date of publication of application:
**16.12.2020   Bulletin 2020/51**

(73) Proprietor: **Sensoriumlab Sp. z o.o.**
**04-703 Warszawa (PL)**

(72) Inventors:
• **ZOLEK, Norbert**
**05-800 Pruszków (PL)**
• **JANUSEK, Dariusz**
**05-075 Warszawa (PL)**
• **MIKLASZEWSKI, Rafa**
**07-111 Wierzbno (PL)**

(74) Representative: **Godlewski, Piotr**
**JWP**
**Rzecznicy Patentowi Dorota Rzazewska sp.k.**
**Sienna Center**
**ul. Zelazna 28/30**
**00-833 Warszawa (PL)**

(56) References cited:
**EP-A1- 0 467 432        WO-A1-88/01485
WO-A1-2015/168157        US-A- 5 606 969
US-A1- 2009 281 413        US-B1- 6 332 091**

**EP 3 749 178 B1**

**Description**

The subject of the invention is a method for measuring pleural effusion, in particular carried out in out-of-hospital conditions.

Pleural effusion is a disease state in which there is an excess of fluid accumulation between the tissue covering the lungs and the inner surface of the chest wall; often co-occurs in patients with cancer, pneumonia, tuberculosis, autoimmune diseases and diseases of the heart, liver and kidneys. This problem affects over 3.4 million people in the United States and Europe each year. In the majority - it is caused by congestive heart failure, bacterial pneumonia, malignant tumors and pulmonary embolism. Also, chronic diseases such as cystic fibrosis may cause pleural effusion.

Pleural effusion occurs when a fluid accumulates between the peritoneal and visceral lung tissue, and the rate of pleural fluid formation exceeds its rate of absorption. This is a serious medical condition and requires prompt medical help - otherwise there is a high probability of life-threatening complications. First of all, an excessive amount of fluid can disrupt the process of breathing and the functioning of the lungs. Massive hydrothorax, due to the lack of specific symptoms, can be a serious threat to life. Statistics show that approximately 30% of patients with pleural effusion die within a year of initial hospitalization [Kookoolis et al., J Pulm Respir Med 2014, 4: 3]. Treatment of the underlying disease is the most common treatment for pleural effusion. Unfortunately, about 15-20% of pleural effusions are still undiagnosed due to their asymptomatic character. Late detection of pleural effusion causes that approximately 2/3 of patients require immediate hospitalization, which additionally creates complications and additional costs for the health care system. Symptoms are more noticeable when there is moderate or high pleural effusion or inflammation is present.

As a standard, pleural effusion is diagnosed in a hospital setting by medical personnel using chest radiography, computed tomography (which requires the use of harmful ionizing radiation) or ultrasonography.

Therefore, non-invasive methods are needed, for example that do not require specialized personnel to use them.

Document US 6332091 B1 discloses a method of detecting pulmonary edema using infrared radiation, especially near infrared. The presence of water in the lungs is detected by measuring the radiation returning to the surface after multiple scattering events within the tissues and comparing its parameters with the calibrated reference values characteristic of edema. The measurement can be made from many sides of the lungs.

From WO 2007061754 A1, a method, a system and an apparatus for measuring the extra-cellular water of a patient are known, wherein the same methodology can also be used to assess the water content at sites located in tissues, on the surface or inside organs such as, for example, lungs, bladder or liver. The measurement method includes the emission of radiation with at least one wavelength directed towards the examined tissue and its detection on the surface at a distance from the point of emission from 1 to 5 cm after internal scattering events. At the same time, radiation of at least one wavelength is directed towards the reference tissue, while part of the emitted radiation is dispersed within the reference tissue, and - as above - the radiation of at least one wavelength away from the reference tissue is detected. Then, the signals are scattered radiation in both the test and reference tissues in order to calculate the extracellular water. Measuring radiation can be in the following wavelength ranges: 950-1400 nm, 1500-1800 nm, and 2000-2300 nm.

In the state of the art, there are also known optical methods for testing various tissue properties in which the analysis of the received signal parameters is carried out using methods from near-infrared spectroscopy. In the indicated systems - the spectral characterization of radiation passing through the tissue is analyzed, in particular the analysis is carried out in terms of changes in the amplitude and phase of the optical signal reaching the detection area.

For example, US 8260389 B2 discloses a method for monitoring bladder function comprising electromagnetic radiation emission, preferably in the range 750 - 950 nm, detection of scattered radiation signals in the volume under study, and their analysis using near infrared spectroscopy (NIRS).

Devices and systems used in such solutions are disclosed, for example, in document US 9498134 B 1. The system proposed there can be used to obtain information such as the presence of hemoglobin or the degree of its oxygenation, while - at the level of the measurement module - it includes a matrix of optical detectors that are arranged at different distances from the corresponding radiation sources.

Document WO2015168157 A1 discloses a method of measuring pleural effusion in which a measuring device containing a radiation source and corresponding radiation detector is applied to the body of the patient being examined in the vicinity of his lungs.

The diagnostic methods described above can be implemented only in hospitals and - additionally - with the participation of qualified medical personnel, such as specialist medical staff or medical technicians. Importantly - according to data published by the World Health Organization (WHO) - it is recognized that still more than 60% of the world population has no access to medical imaging, which is a huge barrier from the point of view of the medical diagnosis itself and, above all, the possibilities and effectiveness of treatment.

Although the described diagnostic methods are well developed in hospitals, the monitoring of outpatients is still an unresolved problem. First of all, the diagnostic medical equipment available on the market - mainly due to its complexity, and often also its dimensions - does not allow monitoring of pleural effusion outside hospital conditions.

The accumulating fluid does not build up evenly, but flows diagonally to the lateral, lower part of the pulmonary plane. This means that without a measurement in the position of at least a sedentary patient, the problem remains determining the position of the fluid level in the pleural cavity and thus the actual quantitative level of effusion. It should be remembered that depending on the inhalation or exhalation, the fluid system in the pleural cavity changes, which depends on the changes in the lung filling through the air and the resulting lung pressure on the fluid accumulated in the pleural cavity.

Recognizing the above-mentioned difficulties, it is an object of the present invention to provide a solution for conducting diagnostics for assessing fluid volume in the pleural cavity in an easy, non-invasive manner and dedicated mainly for out-of-hospital use. Such a solution should assume the ability to measure at home and automatically alert in the event of unacceptable exceeding the level of fluid accumulation in the pleural cavity that would require medical intervention. In order to ensure the reliability of the measurement and to enable its effective use in outpatient settings - the purpose of the invention was to develop a method for monitoring both size and location of the fluid-filled volume.

In the method of measuring the pleural effusion according to the invention, a measuring device containing multiple radiation sources and corresponding radiation detectors is applied to the subject's body near the lower part of his lungs, where radiation sources are adapted to emission and detectors to receive electromagnetic radiation from the near infrared range. The method consists of emission of electromagnetic radiation of at least two wavelengths from the radiation sources. Then intensity of radiation , corresponding to the emitted one and reemitted from the lungs, received at the detector is analysed. At last, the presence and level of pleural effusion is determined, based on the parameters of the change of radiation reemited by the lungs. The method is characterized by the fact that before the emission stage from radiation sources of electromagnetic radiation with at least two wavelengths in the near infrared range, the position of the device within the intercostal space is calibrated, in which electromagnetic radiation of wavelength $\lambda_k$ different from the wavelengths used pleural effusion measurement and corresponding to the maximum bone tissue absorption coefficient. In addition, the method is carried out in the vertical position of the patient being examined.

Preferably, the method is carried out for various instances of inspiration and exhalation of the patient being examined.

The vertical position and moments of inhalation and exhalation of the patient are preferably determined using the indications of the accelerometer system.

It is advantageous if electromagnetic radiation with two wavelengths $\lambda_1$ and $\lambda_2$ is emitted from the near infrared range, where $\lambda_1 \neq \lambda_2$.

Preferably, the wavelength $\lambda_1$ is selected from the range 780 - 850 nm.

It is also preferred that the wavelength $\lambda_2$ is selected in the range of 1000-1150 nm.

Also preferably, when the wavelength $\lambda_k$ is approximately 1200 nm.

According to a preferred solution, the radiation is emitted from a two-dimensional array of nxm radiation sources, whereas it is received in a one-dimensional system nx1 of detectors, where n is the number of rows and m the number of columns in the array of radiation sources.

In addition, it is advantageous if the method is carried out in a reflectance geometry in which the radiation sources and detectors are located on the same side of the lungs on the body of the patient being examined.

Preferably also when the method is implemented in a transmission system in which the radiation sources and detectors are placed on opposite sides of the lungs on the body of the patient being examined.

The main advantages of the proposed solution include shortening the time of hospitalization associated with monitoring the development of pleural effusion, elimination the need for periodic medical examinations after hospitalization, reducing the number of emergency hospitalizations. In addition, early detection of pleural effusion reduces the number of medical complications and the risk of death. In addition, the global cost of healthcare will be significantly reduced, which will be mostly affected by shorter hospitalization, a relatively inexpensive diagnostic method and a reduced risk of complications associated with the late detection of pleural effusion.

The subject of the invention is illustrated in the embodiments in the drawing, in which:

Fig. 1 is a block diagram of a measuring device used for carrying out the method according to the invention,

Fig. 2 is a block diagram of a sensor module in a measuring device,

Fig. 3 is a schematic representation of a disposable, removable overlay for a measuring device,

Fig. 4 is a schematic representation of the location on the body of the measuring device in the reflectance measurement system,

Fig. 5 shows one of the many possible positions of the measuring device for initial calibration to determine the location of the intercostal spaces,

Fig. 6 shows the principle of determining the source - detector pairs involved in the measurement,

Fig. 7 shows the absorption spectrum for various chromophores and water,

Fig. 8 shows the principle of emission and detection of optical radiation in the method according to the invention,

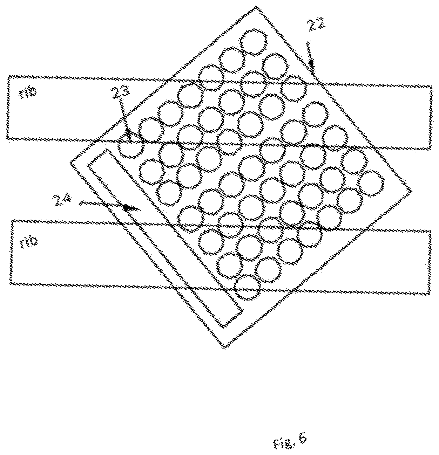

Fig. 6 is a schematic representation of the location of the measuring device in the transmission geometry system.

In the method of pleural effusion measurement according to the invention, a measuring device is used, giving the possibility of its use primarily in out-of-hospital conditions - and therefore portable, autonomous and connected with another device or system for the automated transmission of measurement data to a physician making a decision regarding further diagnostics and / or treatment. The measuring device is equipped with a system of signaling the status of measurement results and to warn whether informing the patient or his environment (not shown) about the existence or absence of a situation associated with the accumulation of fluid in the pleural cavity.

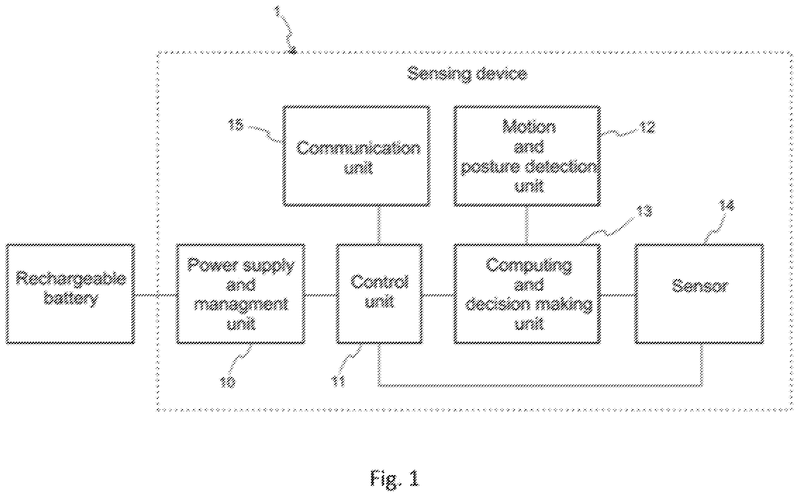

According to fig. 1, the measuring device 1 comprises a power supply and management unit 10, a control module 11, a motion and posture detection module 12, a computation and decision unit 13, a sensor module 14 and a communication module 15. In brief, functions and tasks of specific modules of the measuring device 1 will be described.

The power supply and the management unit 10 serves to extract electricity from the source, process it and deliver it to other electronic modules of the measuring device 1 and is directly connected to a replaceable battery 16 or rechargeable battery. The power supply and management unit 10 cooperates with the control module 11 necessary to control all processes to carry out the method according to the invention as well as provide signaling of the state of measurement results and warning / informing about the occurrence of effusion condition.

The motion and posture detection module 12 is used to acquire and analyze data from devices such as accelerometer, gyroscope or magnetometer (not shown). This module directly cooperates with the computational and decision unit 13, thanks to which the measurement is performed only when the patient is in a vertical position. Measurement data are obtained by means of the computational and decision unit 13, as well as an analysis is carried out and a decision is made to determine the presence or absence of fluid in the pleural cavity.

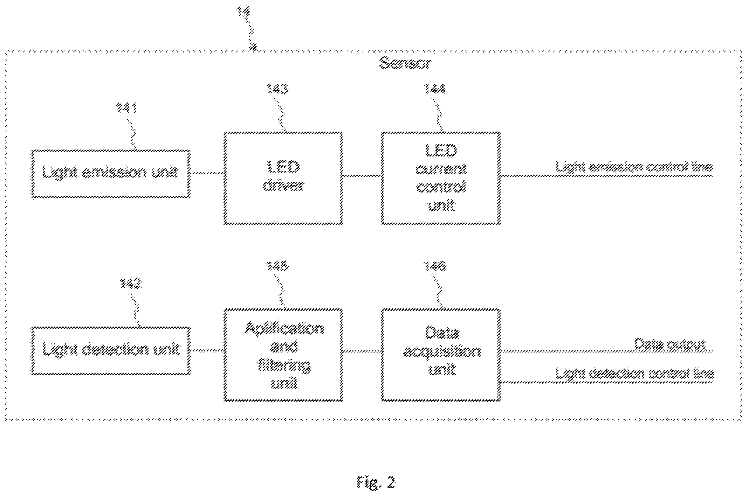

The sensor module 14 in the measuring device 1 is the unit mainly responsible for carrying out the method according to the invention and according to fig. 2 includes a radiation emission module 141, a radiation detection module 142, a LED driver 143, a LED control module 144, an amplifying and filtering unit 145 and the data acquisition module 146. The sensor module 14 is responsible for the implementation of radiation emission from radiation sources, in particular in the near infrared range, the reception in the radiation detector, which has been dispersed and has reached the detection area.

The communication module 15 included in the measuring device 1 is intended for transmitting measurement data to a digital form and its transmission via a radio module, which, for example, may operate in the Bluetooth or GSM standard. Thus, the communication module 15 enables a fully automated transfer of data obtained during the measurement to the physician, who makes a decision on the basis of further diagnosis and / or the need for treatment or use of hospitalization.

In the course of implementing the method according to the invention, the measuring device 1 is applied to the body of the patient being examined, in the vicinity of his lungs. For this purpose, a disposable and replaceable cap 2 made of biocompatible material is fastened to the front part of the measuring device 1 - in which at the same time its sensor module 14 is located. The cap 2 ensures safe application of the measuring device 1 to the patient's body and protection of the measuring area against the influence of external light, which is the source of interference. For this purpose, the overlay material 2 should be characterized by the possibly adhesive front so that the measuring device 1 provided with it can firmly and securely adhere to the patient's skin. In addition, the cover 2 should be made of a dark (black) material having aseptic properties.

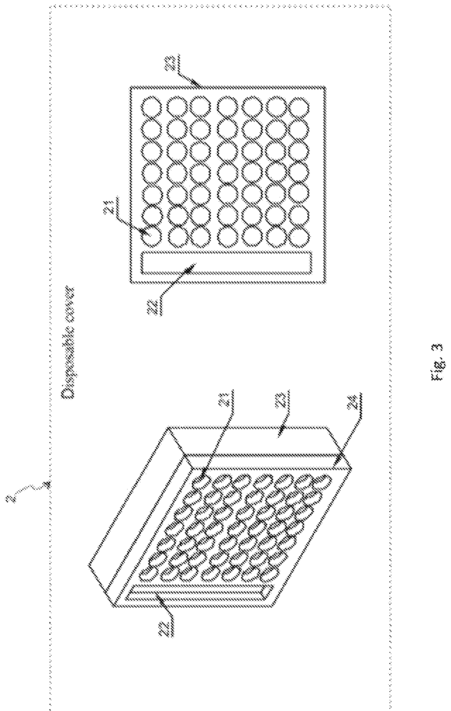

Fig. 3 is a schematic representation of a disposable, replaceable cap 2, mounted on the measuring device 1. Thus, the embodiment of the sensor module 14 is shown in the front part of the measuring device 1 and therefore in direct contact with the cap 2.

As shown in fig. 3, the cap 2 consists of a frame 21 and a front 22 in which holes 23 for radiation sources and an aperture 24 of detectors is made. The openings 23 and 24 made in the cap 2 reflect an implemented circuit of the sensor module 14. Thus, in accordance with the presented embodiment, the radiation emission module 141 is made in the form of a two-dimensional matrix nxm of radiation sources, where n is the number of rows and m number of columns in the matrix. Matrix of radiation sources, in the radiation detection module 142 corresponds to a one-dimensional line nx1 of detectors. Radiation sources are adapted to emission and detectors to receive electromagnetic radiation from the near infrared range.

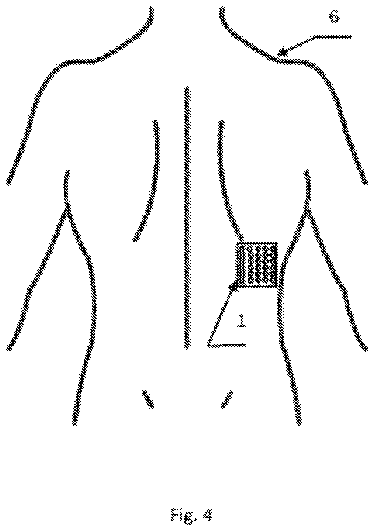

In the method according to the invention, a measuring device 1 is provided to the body of the patient to be examined, in the vicinity of his lungs, provided with a disposable, replaceable cap 2. In the embodiment shown, a measurement in the reflectance system is carried out, which means that the spectral analysis will be subject to diffuse and irradiated radiation, returning to the surface in the detection area. In this case - both the sources and the radiation detectors located in the measuring device 1 are on the same side of the patient's body. According to the embodiment discussed and shown in FIG. 4, the measuring device 1 is applied to the back of the subject.

Before proceeding to the actual measurement, first the calibration of the position of the measuring device 1 is carried out, in which the locations of the intercostal spaces are determined.

The optical properties of the bone can change or distort the measurement result. Therefore, it is important to measure in the intercostal space along the ribs so that there are no bony elements between the place of transmission of the optical signal and the place of its detection by penetration of electromagnetic radiation. Calibration is carried out for a calibration wavelength $\lambda_k$ of, in particular, about 1200 nm, for which the bone absorption factor (16 / cm) [A. N. Bashkatov 2006] is the highest in comparison with the absorption coefficient of other chromophores and water (at most 1 / cm) [Jacques 2013]. In this case - and for the aforementioned calibration wavelength $\lambda_k$ - the decrease in the intensity (attenuation) of electromagnetic radiation mainly affects the bone located between the source and the radiation detector.

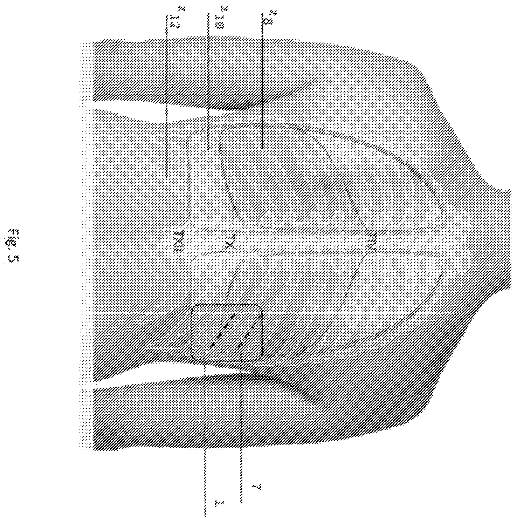

As shown in fig. 5, the calibration method uses the fact that - along the mid axillary line - the lower part of the lungs is most often found on the eighth rib z8, and the pleura on the tenth rib z10. In turn, in the paravertebral line - the lower lung border is on the tenth rib z10 while the pleural border on the twelfth rib z12. Differences in the properties of soft tissues and bones (ribs) allow for precise assessment of the position and direction of the intercostal in relation to the measuring device 1. With respect to the position of the measuring device 1 on the back, in particular the requirement is to locate the measuring device 1 near the lower lung limit at the beginning of the measurement.

The measuring device 1 according to the embodiment allows for a great deal of freedom of its positioning during the measurement in relation to the position of the ribs. At the start of calibration from the radiation sources, electromagnetic radiation with a wavelength $\lambda_k$ of approx. 1200 nm is emitted. Among the set of received signals, pairs of radiation sources - detectors are selected which allow to obtain the largest possible amplitude of the signal, which means that there was no bone in the path between the radiation source and the detector from that pair. Signal amplitude included in the comparison either comes from the radiation source-detector params located at the same distance, or is normalized by the average optical path length or approximately by the emitter-detector distance. The estimation of the optical path length can be carried out with methods of phase change analysis of the transmitted signal known from the literature. Thanks to this information is obtained regarding the specific direction of the radiation source - detector pair, which allows to bypass the bone during the actual measurement. These sets of radiation sources - detectors are then used for proper measurement using different wavelengths to assess the pleural fluid at the level of the given intercostal space. An exemplary arrangement of the measurement device 1 deviated from the vertical on the background of the ribs and the resulting arrangement of the radiation sources and detectors used during the actual measurement is shown in fig. 6.

After the initial calibration of the measuring device 1, the proper measurement of pleural effusion is performed, in which - in the first place - from the radiation sources, radiation pulses in the near infrared range are sequentially emitted. The measurement is carried out in the vertical position of the patient's torso and in various moments of inhalation and exhalation. Data regarding the tested position as well as the inspiratory and expiratory moments are obtained on an ongoing basis from the motion and posture module 12, in particular the values along the vertical and horizontal axes of the accelerometer system (not shown) included in its composition.

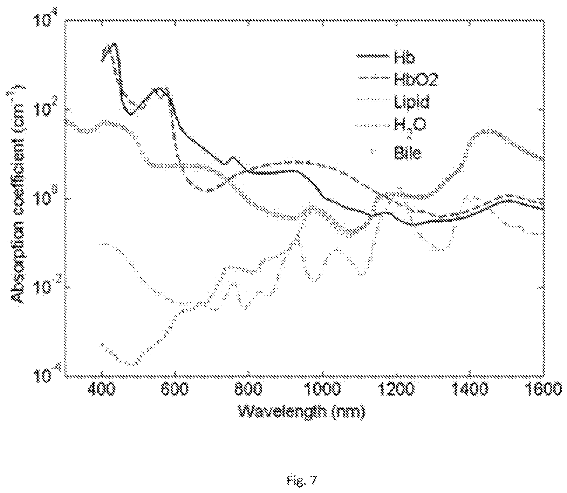

For the purpose of measurement - in the presented embodiment - radiation pulses are emitted with two different wavelengths, i.e. $\lambda_1$ and $\lambda_2$. The wavelengths $\lambda_1$ and $\lambda_2$ are chosen in such a way that the pleural fluid is the absorber mainly only for one wavelength $\lambda_2$, the second wavelength $\lambda_1$ was absorbed mainly by for example hemoglobin and was a reference during measurement allowing to eliminate the individual differences in the obtained absolute values of the signal amplitude measurement. According to the graph in fig. 7, which shows the absorption spectrum for various chromophores and water, the wavelengths $\lambda_1$ and $\lambda_2$ are selected in the following ranges of electromagnetic radiation: $\lambda_1$ in the range of 750 nm to 850 nm and $\lambda_2$ in the range of 1000 nm to 1150 nm.

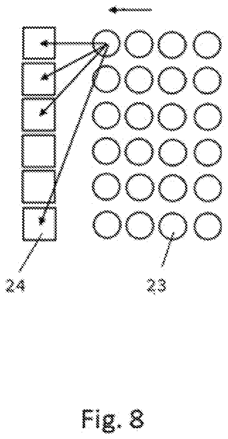

In the next stage, the radiation emitted and dispersed inside the tissues is received in the detectors. The principle of emission and detection of optical radiation in the measurement according to the invention is shown in fig. 8. The radiation pulse emitted from a single source in the matrix row is received in each detector 1...n, which allows obtaining information about the appearance of effusion at this point of the lung. Radiation emission for the remaining and successive in a row of 1...m sources allows for conducting measurements for different depths of electromagnetic radiation penetration with a given wavelength into the tissue under investigation.

After the measurement is completed, in the method according to the invention, the power spectral characteristics received in the signal detector as a function of the mutual location of the radiation source and detector are analyzed, as a result of which the presence and extent of pleural effusion are determined.

Collected measurement data allow to determine the value of radiation intensity in the area between the point of emission and detection.

The change in the concentration of the chromophore (here the pleural fluid) can be obtained using the Lambert Beer law. Change in radiation intensity *Im* relative to the reference intensity *Ir* calculated on the intensity of radiation emitted *Io* at known distances of the radiation source and detector and determined theoretically or on the basis of time travel - optical path length *DP* and known from the literature values of the extinction coefficient allows to get information about changes in $\Delta c$ concentration of an interesting chromophore or pleural fluid:

$$\log(I_0/I_m) - \log(I_0/I_r) = DP \cdot \epsilon \cdot \Delta c$$

The determined values of changes in water absorption for specific and fixed pairs of radiation source - detector allow to determine not only the presence of effusion, but mainly the range of accumulation of fluid in the pleural cavity. Data analysis is carried out in relation to the moments of the inspiratory and expiratory phase obtained from the accelerometer (not shown).

Detection of the presence of an excess amount of pleural fluid or its absence is signaled to the user, in particular in an audible and / or light manner, and then automatically sent to the doctor in order to decide on further therapeutic measures and treatment.

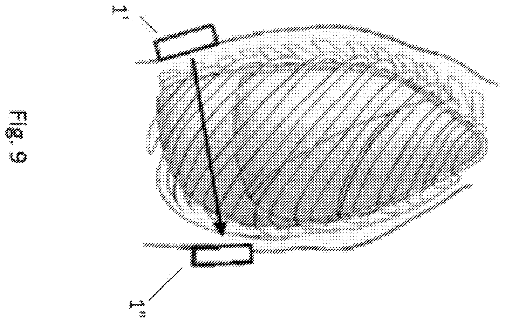

In addition to the described embodiment, in which the method for measuring the pleural effusion according to the invention is carried out in a reflective system - it is possible to modify the measurement to carry it out in the transmission system (fig. 9), which means that the spectral analysis will carried out for signals passing through the pulmonary tissue. In this case - radiation sources 1' and detectors 1" are located on the opposite side of the patient's body near his lungs. In this method, the spectral analysis will be carried out for the signal going through the lung. Plural fluid will give increasing , not decreasing in the radiation intensity. Measurement of pleural effusion performed in the transmission system may be used mainly in neonantological or pediatric applications, i.e. in the case of infants and children, in which the transverse body size is relatively small and allows detection of transmitted radiation at an intensity that allows its proper and effective analysis. Of course, the present invention is not limited to the embodiments shown and various modifications thereof are possible within the scope of the claims without departing from the invention.

## Claims

1. A method of measuring pleural effusion, in which a measuring device (1) containing a large number of radiation sources and corresponding radiation detectors is applied to the body of the patient being examined, in the vicinity of his lungs, where radiation sources are adapted to emission and detectors to receive electromagnetic infrared radiation, in particular near infrared, including the steps comprising:

   - electromagnetic radiation of at least two wavelengths is emitted from the radiation sources,
   - in the detectors, signals constituting electromagnetic radiation re-emitted by the lungs and corresponding to emitted from sources by electromagnetic radiation of at least two wavelengths are recorded, and
   - the spectral characteristics of the signals received in the detector as a function of the difference in radiation intensity for different emitter detector pairs and their location on the body surface are analyzed, wherein before the emission stage from radiation sources of electromagnetic radiation with at least two wavelengths, the position of the measuring device (1) is calibrated to detect the position of the intercostal space, in which electromagnetic radiation of wavelength $\lambda_k$ is emitted and received on the detector a wavelength different from the wavelengths emitted in measuring the pleural effusion and corresponding to the maximum bone tissue absorption coefficient, and wherein the method is carried out in the vertical position of the patient being examined.

2. A method according to claim 1, **characterized in that** it is performed for different moments of inspiration and exhalation of the examined patient.

3. A method according to claim 1 or 2, **characterized in that** the vertical position and moments of inspiration and exhalation of the patient are determined using the indications of the accelerometer system.

4. The method according to claim 1, **characterized in that** electromagnetic radiation with two wavelengths $\lambda_1$ and $\lambda_2$ from the near infrared range is emitted from the radiation sources, where $\lambda_1 \neq \lambda_2$.

5. The method according to claim 4, **characterized in that** the wavelength $\lambda_1$ is selected from the range 780 - 850 nm.

6. The method according to claim 4, **characterized in that** the wavelength $\lambda_2$ is selected from the range of 1000-1150 nm.

7. The method according to claim 1, **characterized in that** the wavelength $\lambda_k$ is about 1200 nm.

8. A method according to any of claims 1 to 7, **characterized in that** the radiation is emitted from a two-dimensional matrix nxm of radiation sources, and it is received by a one-dimensional array of nx1 detectors, where n is the number of rows and m the number of columns in the array of radiation sources.

9. The method according to any one of claims 1 to 8, **characterized in that** it is carried out in a reflectance geometry system in which the radiation sources and detectors are located on the same side of the lungs on the body of the patient being examined.

10. The method according to any one of claims 1 to 8, **characterized in that** it is implemented in a transmission system in which the radiation sources and detectors are located on opposite sides of the lungs on the body of the patient being examined.

## Patentansprüche

1. Ein Verfahren zur Messung des Pleuraergusses, bei dem eine Messvorrichtung (1), die mehrere Strahlungsquellen und entsprechende Strahlungsdetektoren umfasst, am Körper im Bereich der Lungen einer untersuchten Patienten angebracht wird, wobei die Strahlungsquellen zum Senden und die Detektoren zum Empfangen elektromagnetischer Infrarotstrahlung vorgesehen sind, insbesondere im nahen Infrarotbereich, umfassend die folgenden Schritte:

   - Die Strahlungsquellen emittieren elektromagnetische Strahlung mit mindestens zwei Wellenlängen,
   - Signale, die durch die Lunge zurückgestrahlte elektromagnetische Strahlung darstellen, und den von den Quellen ausgestrahlten elektromagnetischen Strahlungen mit mindestens zwei Wellenlängen entsprechen, werden durch die Detektoren erfasst und aufgezeichnet und
   - dann werden die spektralen Eigenschaften der durch Detektoren empfangenen Signale als Funktion der Strahlungsintensitätsdifferenz für verschiedene Sender und Detektor-Paare und deren Position auf der Körperoberfläche analysiert,

   wobei vor der Emission elektromagnetischer Strahlung mit mindestens zwei Wellenlängen durch die Strahlungsquellen die Anordnung der Messvorrichtung (1) am Körper nachjustiert wird, um die Position des interkostalen Raums zu erfassen, in dem elektromagnetische Strahlung der Wellenlänge $\lambda_k$ emittiert und durch einen Detektor eine Wellenlänge empfangen wird, die sich von der bei Messung des Pleuraergusses emittierten Wellenlänge unterscheidet, und die dem maximalen Absorptionskoeffizienten des Knochengewebes entspricht, und wobei das Verfahren in der vertikalen Körperstellung der untersuchten Patienten durchgeführt wird.

2. Ein Verfahren nach Anspruch 1, **dadurch gekennzeichnet, dass** es für verschiedene Phasen der Einatmung und Ausatmung der untersuchten Patienten durchgeführt wird.

3. Ein Verfahren nach Anspruch 1 oder 2, **dadurch gekennzeichnet, dass** die vertikale Stellung und die Phasen der Ein- und Ausatmung des Patienten anhand der Anzeigen am Beschleunigungsmesser bestimmt werden.

4. Ein Verfahren nach Anspruch 1, **dadurch gekennzeichnet, dass** von den Strahlungsquellen elektromagnetische Strahlung mit zwei Wellenlängen $\lambda_1$ und $\lambda_2$ aus dem nahen Infrarotbereich emittiert wird, wobei $\lambda_1 \neq \lambda_2$.

5. Ein Verfahren nach Anspruch 4, **dadurch gekennzeichnet, dass** die Wellenlänge $\lambda_1$ aus dem Bereich 780 - 850 nm ausgewählt wird.

6. Ein Verfahren nach Anspruch 4, **dadurch gekennzeichnet, dass** die Wellenlänge $\lambda_2$ aus dem Bereich von 1000-1150 nm ausgewählt wird.

7. Ein Verfahren nach Anspruch 1, **dadurch gekennzeichnet, dass** die Wellenlänge $\lambda_k$ etwa 1200 nm betragen soll.

8. Ein Verfahren nach einem der Ansprüche 1 bis 7, **dadurch gekennzeichnet, dass** die Strahlung von einer zweidimensionalen nxm-Matrix der Strahlungsquellen emittiert und durch eine eindimensionale Anordnung der nxl-Detektoren empfangen wird, wobei n die Anzahl der Zeilen und m die Anzahl der Spalten der Anordnung der Strahlungsquellen ist.

**9.** Ein Verfahren nach einem der Ansprüche 1 bis 8, **dadurch gekennzeichnet, dass** es in einem System mit Reflexionsgeometrie durchgeführt wird, bei dem die Strahlungsquellen und die Detektoren auf der gleichen Seite der Lunge am Körper des untersuchten Patienten angebracht werden.

**10.** Ein Verfahren nach einem der Ansprüche 1 bis 8, **dadurch gekennzeichnet, dass** es in einem Übertragungssystem durchgeführt wird, bei dem die Strahlungsquellen und Detektoren auf gegenüberliegenden Seiten der Lunge am Körper des untersuchten Patienten angebracht werden.

## Revendications

**1.** Procédé de mesure de l'épanchement pleural, dans lequel un dispositif (1) de mesure contenant un grand nombre de sources de rayonnement et des détecteurs de rayonnement correspondants est appliqué sur le corps du patient examiné, à proximité de ses poumons, où les sources de rayonnement sont adaptées à émission et détecteurs pour recevoir un rayonnement électromagnétique infrarouge, en particulier proche infrarouge, comprenant les étapes comprenant:

- un rayonnement électromagnétique d'au moins deux longueurs d'onde est émis par les sources de rayonnement
- dans les détecteurs, on enregistre des signaux constituant des rayonnements électromagnétiques réémis par les poumons et correspondant à des rayonnements électromagnétiques d'au moins deux longueurs d'onde émis par des sources, et
- les caractéristiques spectrales des signaux reçus dans le détecteur en fonction de la différence d'intensité du rayonnement pour différentes paires émetteur-détecteur et leur emplacement sur la surface du corps sont analysées,

dans lequel, avant l'étape d'émission par des sources de rayonnement d'un rayonnement électromagnétique ayant au moins deux longueurs d'onde, la position du dispositif de mesure (1) est étalonnée pour détecter la position de l'espace intercostal, dans lequel un rayonnement électromagnétique de longueur d'onde $\lambda_k$ est émis et reçu sur le détecteur, la longueur d'onde différente des longueurs d'onde émises lors de la mesure de l'épanchement pleural et correspondant au coefficient d'absorption maximal du tissu osseux, et dans lequel le procédé est mis en oeuvre dans la position verticale du patient examiné.

**2.** Procédé selon la revendication 1, **caractérisé en ce qu'il** est réalisée pour différents moments d'inspiration et d'expiration du patient examine.

**3.** Procédé selon la revendication 1 ou 2, **caractérisé en ce que** la position verticale et les moments d'inspiration et d'expiration du patient sont déterminés en utilisant les indications du système accélérométrique.

**4.** Procédé selon la revendication 1, **caractérisé en ce qu'**un rayonnement électromagnétique de deux longueurs d'onde $\lambda_1$ et $\lambda_2$ du domaine de l'infrarouge proche est émis par les sources de rayonnement, où $\lambda_1 \neq \lambda_2$.

**5.** Procédé selon la revendication 4, **caractérisé en ce que** la longueur d'onde $\lambda_1$ est choisie dans la plage 780 - 850 nm.

**6.** Procédé selon la revendication 4, **caractérisé en ce que** la longueur d'onde $\lambda_2$ est choisie dans la plage 1000-1150 nm.

**7.** Procédé selon la revendication 1, **caractérisé en ce que** la longueur d'onde $\lambda_k$ est d'environ 1200 nm.

**8.** Procédé selon l'une quelconque des revendications 1 à 7, **caractérisé en ce que** le rayonnement est émis par une matrice bidimensionnelle nxm de sources de rayonnement, et il est reçu par un réseau unidimensionnel de nxl détecteurs, où n est le nombre de rangées et m le nombre de colonnes dans le réseau de sources de rayonnement.

**9.** Procédé selon l'une quelconque des revendications 1 à 8, **caractérisé en ce qu'il** est mis en oeuvre dans un système de géométrie de réflectance dans lequel les sources de rayonnement et les détecteurs sont situés du même côté des poumons sur le corps du patient examiné.

**10.** Procédé selon l'une quelconque des revendications 1 à 8, **caractérisé en ce qu'il** est mis en œuvre dans un système de transmission dans lequel les sources de rayonnement et les détecteurs sont situés sur des côtés opposés des poumons sur le corps du patient examiné.

Fig. 1

Sensing device

- Motion and posture detection unit — 12
- Communication unit — 15
- Computing and decision making unit — 13
- Control unit — 11
- Sensor — 14
- Power supply and management unit — 16
- Rechargeable battery

Fig. 2

Fig. 3

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6332091 B1
- WO 2007061754 A1
- US 8260389 B2
- US 9498134 B1
- WO 2015168157 A1

**Non-patent literature cited in the description**

- **KOOKOOLIS et al.** *J Pulm Respir Med,* 2014, vol. 4, 3